# EUROPEAN PATENT APPLICATION

(11) **EP 4 389 876 A1**
(43) Date of publication of application: **26.06.2024**
(21) Application number: 23755921.6
(22) Date of filing: 21.02.2023
(51) Int. Cl.: C12N 5/071

(54) **METHOD FOR PREPARING RENAL PODOCYTES BY DISCONTINUOUS DIFFERENTIATION**

(30) Priority: 21.02.2022 CN 202210155136
(71) Applicant: Guangzhou Asia Kidney Rebuilding Medical Technology Ltd., Guangzhou, Guangdong 510700 (CN)
(72) Inventor: WANG, Heng, Guangzhou, Guangdong 510700 (CN); ZHENG, Lixin, Guangzhou, Guangdong 510700 (CN); QIU, Jiang, Guangzhou, Guangdong 510700 (CN); CHEN, Jiaxin, Guangzhou, Guangdong 510700 (CN); LIN, Kaixin, Guangzhou, Guangdong 510700 (CN)
(74) Representative: Kehl, Ascherl, Liebhoff & Ettmayr Patentanwälte Partnerschaft mbB
(86) International application number: PCT/CN2023/077430
(87) International publication number: WO 2023/155921

(57) **Abstract**

The present invention provides a method for preparing renal podocytes by discontinuous differentiation. Induced pluripotent stem cells are differentiated into the renal podocytes by using a discontinuous method. According to the method, pluripotent stem cells can be subjected to targeted induction of differentiation to obtain renal progenitor cells; renal progenitor cells with a high degree of differentiation are identified and cryopreserved, and then are thawed and subjected to targeted induction of differentiation into podocytes after large-scale preparation. The podocytes obtained by this method have high differentiation efficiency, high consistency, and a high survival rate.

## Description

### TECHNICAL FIELD

The present disclosure belongs to the technical field of cell biology, and in particular relates to a method for preparing renal podocytes by discontinuous differentiation.

### BACKGROUND

Podocytes are terminally differentiated inherent renal epithelial cells, which possess no proliferative ability and are difficult to be cultured in vitro in principle. People initially used other renal cell lines to study the pathogenesis of podocytopathies. In 1970s, a method for obtaining primary podocytes by isolating glomeruli in vivo was established, so that people can directly study on podocytes. Since the primary cells cannot be proliferated and passaged, the number of cells obtained from each primary culture is limited, which restricts the application of primary podocytes. In view of these limitations, podocyte cell lines have been established through immortalization techniques. Although the problem of cell number limitation of primary podocytes can be overcome with established podocyte cell lines, the expressions of podocyte markers in the immortalized podocyte lines are unstable, and different podocyte lines even from the same species vary greatly. Therefore, obtaining podocytes with stable phenotype has great significance in understanding and treating kidney diseases.

With the research progress on kidney development, researchers have been able to differentiate pluripotent stem cells into renal organoid cells or renal cells. Generally, Pluripotent stem cells are induced to differentiate to a primitive streak stage by a WNT agonist, and then to a mesoderm stage, and finally to renal organoids. There are some methods to differentiate pluripotent stem cells into podocytes by using variable inducible factors and culture medium to induce big changes in cell fates. It costs massive capital and time for successful induction yet the differentiated podocytes are largely varied in different batches. Therefore, it is necessary to improve the method for differentiation into podocytes from pluripotent stem cells. In the patent (CN110662832A), methods for inducing differentiation to nephron progenitor cells from intermediate mesodermal cells and pluripotent stem cells, respectively, have been shown. In the above patent, it is necessary to use many reagents and to change different medium with different components every day. CHIR99021 (a GSK-3β inhibitor) is essential in entire differentiation process which takes a long time for about 11 days. The published paper (Yoshimura Y, et al. Manipulation of Nephron-Patterning Signals Enables Selective Induction of Podocytes from Human Pluripotent Stem Cells [J]. Journal of the American Society of Nephrology, 2019, 30(2): ASN.2018070747) established a method for inducing podocyte from nephron progenitor cells, which needs multiple reagents as well and takes 6-8 days. According to the available technology aforementioned, it generally takes a total of 17-20 days for inducing podocytes from pluripotent stem cells through nephron progenitor cell stage. There are other methods for podocytes differentiation from pluripotent stem cells which take a shorter time as 13 days. However, these methods are all without going through the intermediate nephron progenitor cells stage, which is a continuous differentiation process, and has high requirements on experimental operation and cell morphology identification.

### SUMMARY

The present disclosure intends to simplify the process of differentiation into renal podocytes from pluripotent stem cells and provides a method for preparing podocytes (by inducing differentiation) which is simple and can undergo a continuous differentiation process into podocytes from pluripotent stem cells in one or two separate steps. The first step is to obtain nephron progenitor cells by stable induction and the differentiated nephron progenitor cells can be well cryopreserved. The second step is to induce nephron progenitor cells to podocytes. Therefore the process of inducing podocytes can be divided into two discontinuous processes.

One objective of the present disclosure is to provide a method for preparing podocytes from pluripotent stem cells.

Another objective of the present disclosure is to provide a method for preparing nephron progenitor cells from pluripotent stem cells.

Still another objective of the present disclosure is to provide a method for preparing podocytes from nephron progenitor cell.

Still another objective of the present disclosure is to provide a method for discontinuous differentiation of pluripotent stem cells into podocytes.

Still another objective of the present disclosure is to provide a podocyte.

Still another objective of the present disclosure is to provide a culture medium.

The present disclosure is implemented through the following technical solutions:
Provided is a method for preparing podocytes from pluripotent stem cells, including the following steps:
S1: inducing pluripotent stem cells to an intermediate mesoderm stage using a culture medium containing a GSK-3α/β inhibitor;
S2: inducing the cells obtained in step S1 to differentiate into nephron progenitor cells using a culture medium containing FGF9, BMP7 and vitamin A; and
S3: inducing the nephron progenitor cells obtained in step S2 to differentiate into podocytes using a culture medium containing fetal bovine serum, Glutamax-1, vitamin D3 and vitamin A.

In the present disclosure, the culture medium containing the GSK-3α/β inhibitor is first used to induce pluripotent stem cells to differentiate to the intermediate mesoderm stage, and then an inducing culture medium containing FGF9, BMP7 and vitamin A is used to stimulate some signaling pathways which associated with kidney development causing the cells in the intermediate mesoderm stage to directly differentiate into nephron progenitor cells. Before nephron progenitor cells inducing into podocytes, fetal bovine serum is added to the culture medium to reduce the dosage of cytotoxic vitamin A. The addition of fetal bovine serum hence improves the survival rate of podocytes.

The above-mentioned pluripotent stem cells are human induced pluripotent stem cells.

Preferably, in step S1, the GSK-3α/β inhibitor is CHIR99021, and a working concentration of the CHIR99021 is 1 - 100 µM; in step S2, a working concentration of the FGF9 is 50 - 500 ng/mL, a working concentration of the BMP7 is 50 - 500 ng/mL, and a working concentration of the vitamin A is 50 - 500 nM; and in step S3, a volume fraction of the fetal bovine serum is 5% - 30%, a volume fraction of the Glutamax-1 is 0.1% - 10%, a working concentration of the vitamin D3 is 50 - 500 nM, and a working concentration of the vitamin A is 50 - 500 nM.

The specific duration of cell culture involves: for induction in step S1, a culture time is 65 - 96 hours; for inducing the cells obtained in step S1 to differentiate into nephron progenitor cells in step S2, a culture time is 42 - 72 hours; and for inducing the nephron progenitor cells obtained in step S2 to differentiate into podocytes in step S3, a culture time is at least 192 hours, preferably 192 - 288 hours. Preferably, the culture medium is changed once every 16 - 36 hours.

Preferably, a seeding density of the pluripotent stem cells in step S1 is 1×10⁵ - 1×10⁶ cells/10 cm².

As a preferred embodiment, the culture medium in step S1 is Advanced RPMI-1640 medium; the culture medium in step S2 is Advanced RPMI-1640 medium; and the culture medium in step S3 is DMEM/F12 medium.

Preferably, before inducing the nephron progenitor cells to differentiate into podocytes in step S3, the nephron progenitor cells are transferred to a collagen-coated culture vessel, wherein the collagen is type I collagen, and the culture vessel is a culture dish, a culture plate, or a culture flask. Preferably, a seeding density of the nephron progenitor cells is 5×10⁶ - 5×10⁷ cells/10 cm².

Preferably, before inducing the pluripotent stem cells to an intermediate mesoderm stage using a culture medium containing a GSK-3α/β inhibitor in step S1, the pluripotent stem cells are cultured in a culture medium containing a ROCK inhibitor, preferably Y27632. Preferably, the working concentration of Y27632 is 5 - 30 µM, and the culture time is preferably 20 - 36 hours.

Provided is a method for preparing nephron progenitor cells from pluripotent stem cells including the following steps:
inducing pluripotent stem cells to an intermediate mesoderm stage using a culture medium containing a GSK-3α/β inhibitor; and
inducing the cells in the intermediate mesoderm stage to differentiate into nephron progenitor cells using a culture medium containing FGF9, BMP7 and vitamin A.

Provided is a method for preparing podocytes from nephron progenitor cells, in which the nephron progenitor cells are induced to differentiate into the podocytes using a culture medium containing fetal bovine serum, Glutamax-1, vitamin D3 and vitamin A.

Specifically, the nephron progenitor cells are human-derived nephron progenitor cells. Preferably, a seeding density of the nephron progenitor cells is 5×10⁶ - 5×10⁷ cells/10 cm².

Preferably, the culture medium is DMEM/F12 medium, a volume fraction of the fetal bovine serum is 5% - 30%, a volume fraction of the Glutamax-1 is 0.1% - 10%, a working concentration of the vitamin D3 is 50 - 500 nM, and a working concentration of the vitamin A is 50 - 500 nM.

Preferably, the culture time for differentiation is at least 192 hours, preferably 192 - 288 hours. Preferably, the cell culture medium is changed once every 16 - 36 hours.

Preferably, the nephron progenitor cells are cultured in a collagen-coated culture vessel. Preferably, the collagen is type I collagen, and the preferred culture vessel is a culture dish, a culture plate, or a culture flask.

Provided is a method for discontinuous differentiation of pluripotent stem cells into podocytes, including the following steps:
S1: inducing pluripotent stem cells to an intermediate mesoderm stage using a culture medium containing a GSK-3α/β inhibitor;
S2: inducing the cells obtained in step S1 to differentiate into nephron progenitor cells using a culture medium containing FGF9, BMP7 and vitamin A;
S3: cryopreserving the nephron progenitor cells obtained in step S2;
S4: recovering the nephron progenitor cells cryopreserved in step S3; and
S5: inducing the nephron progenitor cells recovered in step S4 to differentiate into podocytes using a culture medium containing fetal bovine serum, Glutamax-1, vitamin D3 and vitamin A.

The above-mentioned pluripotent stem cells are human induced pluripotent stem cells.

Preferably, in step S1, the GSK-3α/β inhibitor is CHIR99027, and a working concentration of the CHIR99021 is 1 - 100 µM; in step S2, a working concentration of the FGF9 is 50 - 500 ng/mL, a working concentration of the BMP7 is 50 - 500 ng/mL, and a working concentration of the vitamin A is 50 - 500 nM; and in step S5, a volume fraction of the fetal bovine serum is 5% - 30%, a volume fraction of the Glutamax-1 is 0.1% - 10%, a working concentration of the vitamin D3 is 50 - 500 nM, and a working concentration of vitamin A is 50 - 500 nM.

The specific duration of cell culture involves: for inducing in step S1, a culture time is 65 - 96 hours; for inducing the cells obtained in step S1 to differentiate into nephron progenitor cells in step S2, a culture time for differentiation is 42 - 72 hours; and for inducing the nephron progenitor cells recovered in step S4 to differentiate into podocytes in step S5, a culture time for differentiation is at least 192 hours, preferably 192 - 288 hours. Preferably, the cell culture medium is changed once every 16 - 36 hours.

As a preferred embodiment, the culture medium in step S1 is Advanced RPMI-1640 medium; the culture medium in step S2 is Advanced RPMI-1640 medium; and the culture medium in step S3 is DMEM/F12 medium.

As a preferred embodiment, a method for recovering in step S4 includes: coating a culture vessel with type I collagen and incubating in an incubator for at least 1 hour for later use. After recovery, the cells cryopreserved in step S3 are transferred to the type I collagen-coated culture vessel. The culture vessel includes a culture dish, a culture plate, or a culture flask. Preferably, a seeding density of the nephron progenitor cells is 5×10⁶ - 5×10⁷ cells/10 cm².

Preferably, before inducing the pluripotent stem cells to an intermediate mesoderm stage using a culture medium containing a GSK-3α/β inhibitor in step S1, the pluripotent stem cells are cultured in a culture medium containing a ROCK inhibitor. Preferably, the ROCK inhibitor is 5 - 30 µM Y27632, and the culture time is preferably 20 - 36 hours.

The present disclosure provides a podocyte prepared by any one of the above methods.

The present disclosure provides a culture medium containing 5% - 30% volume fraction of fetal bovine serum, 0.1% - 10% volume fraction of Glutamax-1, 50 - 500 nM vitamin D3, and 50 - 500 nM vitamin A.

The technical solution of the present disclosure has the following beneficial effects:
(1) The process to induce differentiation of pluripotent stem cells into podocytes is easy to operate and it takes a short time in whole differentiation cycle;
(2) Only a few reagents are needed; the induction time is short; the differentiation rate is high, and the cost is low;
(3) The obtained podocytes are at high survival rate, in a large number, and can apply to large-scale production; and
(4) The nephron progenitor cells can be obtained in the process of differentiation, and can be cryopreserved, which is helpful to conduct experiments in stages.

### BRIEF DESCRIPTION OF DRAWINGS

The present disclosure will be further described below with attached drawings and examples, wherein:
Figure 1 is a diagram illustrating mRNA relative expression levels of the genes *OCT4, NANGO* and *PAX2* in the human induced pluripotent stem cells of Example 1 and the nephron progenitor cells obtained in step S2.
Figure 2 is an immunofluorescence image of proteins WT1 and PAX2 in the nephron progenitor cells obtained in step S2 of Example 1.
Figure 3 is a diagram illustrating the morphology of the cell culture on days 1-4 after step S5 of Example 1.
Figure 4 is an RNA-sequencing result graph of the human induced pluripotent stem cells, and the nephron progenitor cells obtained in step S2 and the podocytes obtained in step S5 of Example 1.
Figure 5 is an immunofluorescence image of podocyte marker proteins Synaptopodin and Nephrin of the podocytes obtained in step S5 of Example 1.
Figure 6 is a diagram illustrating the morphology of the cell culture on days 1-4 after step S3 of Example 2.
Figure 7 is an immunofluorescence image of podocyte marker proteins Synaptopodin and Nephrin of the podocytes obtained in step S3 of Example 2.
Figure 8 is diagram illustrating the morphology of the cell culture on days 1-4 after step S5 of Comparative Example 1.
Figure 9 is a diagram illustrating mRNA relative expression levels of the genes *OCT4, NANGO, PAX2* and *WT1* in the human induced pluripotent stem cells and the cells obtained in step S2 of Comparative Example 2.
Figure 10 is a diagram illustrating mRNA relative expression levels of the genes *Nephrosis2, Synaptopodin* and *Nephrin* in the human induced pluripotent stem cells and the cells obtained in step S2 of Comparative Example 3.

### DETAILED DESCRIPTION OF EMBODIMENTS

The conception of the present disclosure and the technical effects produced by the present disclosure will be clearly and completely described below with reference to examples, so as to fully understand the objectives, features and effects of the present disclosure. Obviously, the described embodiments are only a part of embodiments of the present disclosure, not all of it. Based on the embodiments of the present disclosure, all other embodiments obtained by those skilled in the art without creative efforts shall fall within the protection scope of the present disclosure.

In the following examples and comparative examples, a method for preparing a single layer of human induced pluripotent stem cells includes the following process.

Matrigel is diluted with DMEM/F12 medium at a ratio of 1: 20, then 1 mL of diluted Matrigel is added to each well of a 6-well plate; after the bottom of the well plate is evenly covered with Matrigel, the six-well plate is incubated in a 37 °C incubator for 1 hour. Human induced pluripotent stem cells are digested and dissociated into single-cell suspension using 500 µL of Accutase enzyme. Digestion is terminated by adding 5 mL of mTeSR1 medium; the cell suspension is centrifuged at a speed of 200×g for 4 min. The supernatant is removed and 1 mL of mTeSR1 medium is added to resuspend the cells; the human induced pluripotent stem cells are seeded in the 6-well plate covered with Matrigel at a density of 1×10⁵ cells/well. The ROCK inhibitor, Y27632, is added with a working concentration of 10 µM, and the cells are cultured for 24 hours so as to prepare a single layer of human induced pluripotent stem cells.

In the following examples and comparative examples, primers used for qPCR are shown in Table 1.

**Table 1**

| Genes | Forward primers and reverse primers | Primer sequences |
|---|---|---|
| *NANOG* | *F* | *TGTTTGGGATTGGGAGGCTT* (SEQ ID NO: 1) |
| | *R* | *GCACAACCAACAAATTAGGGGA* (SEQ ID NO: 2) |
| *Pax2* | *F* | *TGAGTTTGAGAGGCGACACG* (SEQ ID NO: 3) |
| | *R* | *TGCAGTGCATATCCATGGGG* (SEQ ID NO: 4) |
| *Wt1* | *F* | *CACGGTGTCTTCAGAGGCAT* (SEQ ID NO: 5) |
| | *R* | *ACACATGAAGGGGCGTTTCT* (SEQ ID NO: 6) |
| *Synaptopodin* | *F* | *TCTACCATGGCTACCTGCCT* (SEQ ID NO: 7) |
| | *R* | *GGCAGGCTCCTTGATAGGTG* (SEQ ID NO: 8) |
| *OCT4* | *F* | *CTTGAATCCCGAATGGAAAGGG* (SEQ ID NO: 9) |
| | *R* | *GTGTATATCCCAGGGTGATCCTC* (SEQ ID NO: 10) |
| *Nephrin* | *F* | *TCACCGTGAATGTTCTGTTCC* (SEQ ID NO: 11) |
| | *R* | *AGTGTGGCTAAGGGATTACCC* (SEQ ID NO: 12) |
| *Nephrosis2* | *F* | *GTGGTGGACGTGGATGAGG* (SEQ ID NO: 13) |
| | *R* | *CCGGAGGATTTGGTACCTTCC* (SEQ ID NO: 14) |

### Example 1

In this example, podocytes were prepared from human pluripotent stem cells (by discontinuous differentiation), including the following process.

S 1: A single layer of pluripotent stem cells were continuously induced for 3 days using Advanced RPMI-1640 medium containing 5 µM CHIR99027 as the initiating culture medium, and the culture medium was changed every 24 hours.

S2: The cells were cultured for another 2 days using Advanced RPMI-1640 medium containing 200 ng/mL FGF9, 50 ng/mL BMP7 and 100 nM vitamin A as the inducing culture medium, and the culture medium was changed every 24 hours.

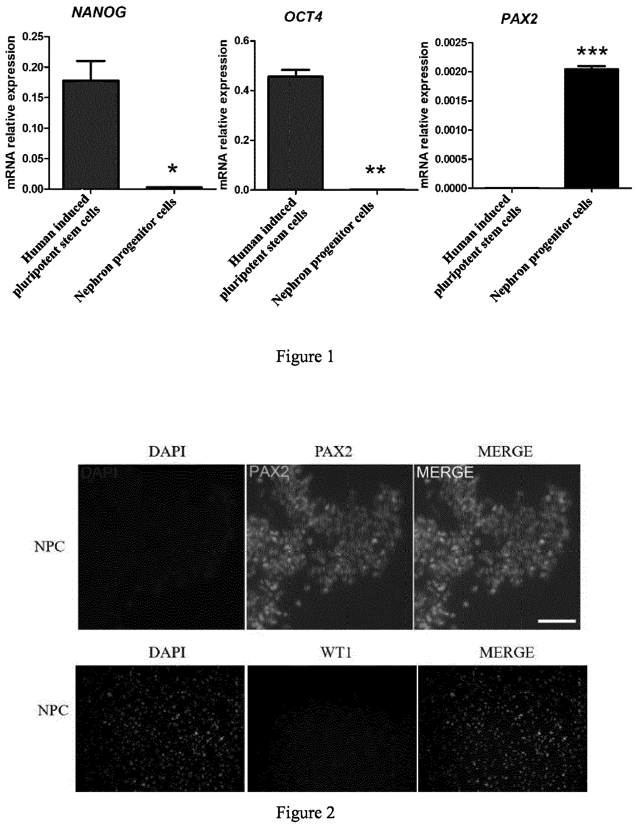

The human induced pluripotent stem cells and some of the cells obtained in S2 were collected to validate the gene expressions of *OCT4, NANOG* and *PAX2* by qPCR, and measured the protein expressions of WT1 and PAX2 of nephron progenitor cells by immunofluorescence. The results are shown in Figure 1 and Figure 2, respectively.

S3: The cells (nephron progenitor cells) obtained in S2 were cryopreserved by the following process: discarding cell culture medium, gently rinsing the cells with 1 mL of PBS without calcium ions and magnesium ions at room temperature, adding 1ml/well Accutase enzyme to the plate, digesting in a 37 °C incubator for 2 minutes, then gently blowing the digested cells off from the bottom of the plate using a pipette, transferring 1 mL of cell suspension to a 15 mL centrifuge tube, adding 10 mL of DMEM/F12 medium containing 10% (v/v) fetal bovine serum, gently and evenly mixing, then centrifuging at a speed of 200×g for 4 minutes at room temperature, discarding the supernatants, resuspending the cell pellet with a cell cryopreservation solution, then transferring the cells to a cryopreservation tube, placing the cells in a -80 °C refrigerator after gradient freezing, and then transferring the cells to a liquid nitrogen tank for cryopreservation on the next day.

S4: 1 mL of type I collagen was added for coating the surface of a 6-well plate, and the plate was incubated in a 37 °C incubator for at least 1 hour for later use. The cells cryopreserved in S3 were taken out from the liquid nitrogen tank and quickly recovered in a 37 °C water bath. The cell suspension was transferred to a 15 mL centrifuge tube. 10 mL of DMEM/F12 medium containing 20% (v/v) fetal bovine serum was added, and then the obtained mixture was centrifuged at 200×g for 4 minutes at room temperature.

S5: The cells was resuspended with DMEM/F12 medium containing 20% (v/v) fetal bovine serum and Glutamax-1 (100×), the cells were seeded in the 6-well plate coated with type I collagen at a density of 5×10⁶ cells/well after the excessive type I collagen was removed, and vitamin D3 with a working concentration of 100 nM and vitamin A with a working concentration of 100 nM were added. The culture medium was changed every 24 hours, and the cells were cultured continuously for 8 days.

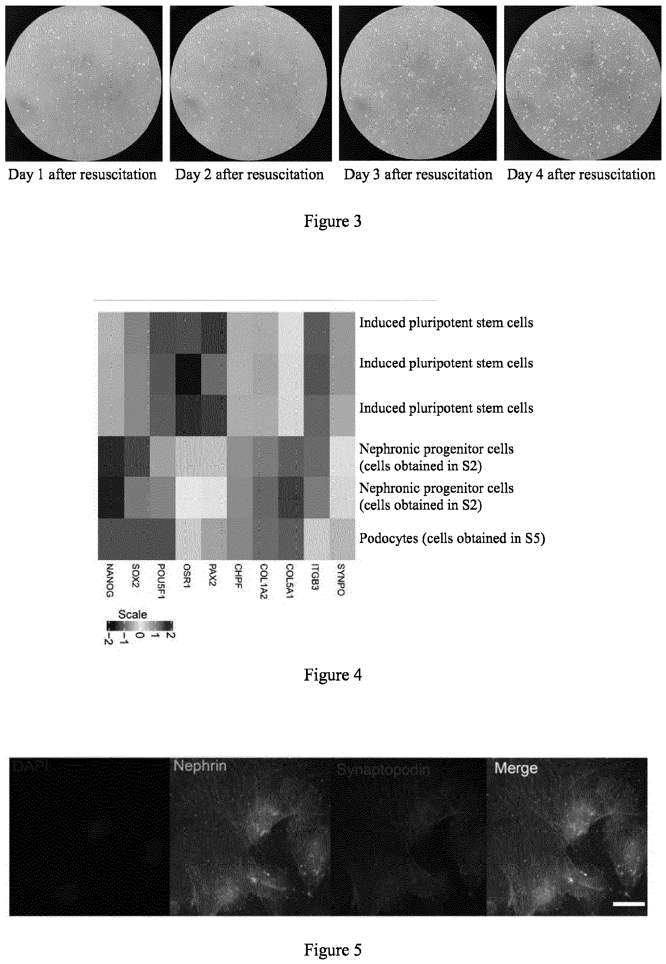

The morphology cells on days 1-4 after recovery is shown in Figure 3, showing that the recovered cells were proliferative, in good condition and clear shape. After reaching confluence, the cells population had defined boundaries and the cells tightly connected to each other.

Total RNA from the human induced pluripotent stem cells (iPSCs), the cells (nephron progenitor cells, NPCs) obtained in step S2 and the cells (podocytes) obtained in step S5 was extracted respectively, then magnetic beads with Oligo(dT) were used to enrich mRNAs, and Fragmentation Buffer was added to break the mRNAs into shorter fragments. A first strand of cDNA was synthesized by using an mRNA fragment as a template and random hexamers. Buffer, dNTPs, RNase H and DNA polymerase I were altogether added to synthesize a second strand of cDNA. Target fragments were recovered by purifying with a PCR kit and eluting with buffer, and subjecting to terminal repair, addition of base A and sequencing linkers, and then agarose gel electrophoresis for fragments separation. A library was established by PCR amplification, and finally sequencing was performed using Illumina HiSeq2000. In addition, the podocyte markers Synaptopodin and Nephrin of the cells obtained in step S5 were measured by immunofluorescence.

Results: as showed by RNA-sequencing experiments, the induced pluripotent stem cells highly expressed corresponding marker genes *NANOG, SOX2* and *POU5F1*; the cells obtained in step S2 highly expressed corresponding marker genes *OSR1* and *PAX2*, proving the successful differentiation into nephron progenitor cells; and the cells obtained in step S5 highly expressed corresponding marker genes *SYNPO* and *ITGB3,* proving the successful differentiation into podocytes (Figure 4). Immunofluorescence images showed that the PAX2 and WT1 proteins that were induced to differentiate in step S2 were widely expressed in the cells (Figure 2); and Synaptopodin and Nephrin that were induced to differentiate in step S5 were also widely expressed in the cells (Figure 5). The results of immunofluorescence and RNA-sequencing experiments demonstrated that the cells in step S2 had differentiated into nephron progenitor cells, and the cells in step S5 had differentiated into podocytes.

### Example 2

In this example, podocytes were prepared by the following specific process.

S1: A single layer of pluripotent stem cells were continuously induced for 3 days using Advanced RPMI-1640 medium containing 10 µM CHIR99027 as the initiating culture medium, and the culture medium was changed every 24 hours.

S2: The cells were cultured for another 2 days using Advanced RPMI-1640 medium containing 100 ng/mL FGF, 100 ng/mL BMP7 and 200 nM vitamin A as the inducing culture medium, and the culture medium was changed every 24 hours.

S3: The cells were digested with trypsin and then centrifuged. After discarding the supernatants, the cell pellet was resuspended with DMEM/F12 medium containing 20% (v/v) fetal bovine serum and Glutamax-1 (100×). The cells were seeded in a 6-well plate coated with type I collagen at a density of 5×10⁶ cells/well, and vitamin D3 with a working concentration of 200 nM and vitamin A with a working concentration of 200 nM were both added. The culture medium was changed every 24 hours, and the cells were cultured continuously for 8 days.

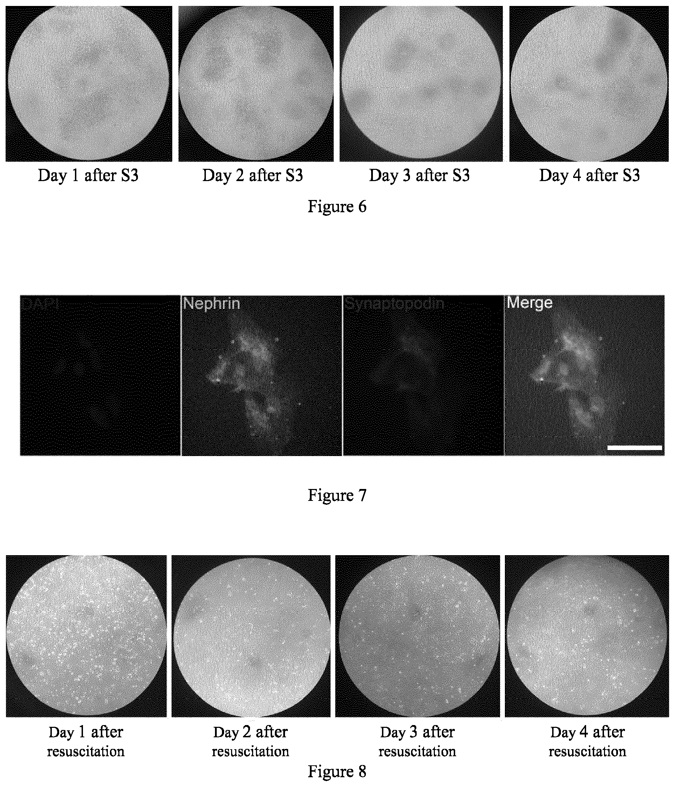

The morphology of cells on days 1-4 after step S3 is shown in Figure 6, showing that the cells were proliferative, in good condition and clear shape. After reaching confluence, the cells population had defined boundaries and the cells tightly connected to each other.

The podocyte markers Synaptopodin and Nephrin of the cells obtained in step S3 were measured by immunofluorescence.

Results: immunofluorescence images (Figure 7) showed that the Synaptopodin and Nephrin that were induced to differentiate in step S3 were widely expressed in the cells. The results of immunofluorescence experiments proved that the cells in step S3 had differentiated into podocytes.

### Comparative Example 1

The method for preparing podocytes in this comparative example was different from that in Example 1 in the aspect of cryopreservation time, with the cells being cryopreserved one day in advance. The specific process was as follows.

S 1: A single layer of pluripotent stem cells were continuously induced for 3 days using Advanced RPMI-1640 medium containing 5 µM CHIR99027 as the initiating culture medium, and the culture medium was changed every 24 hours.

S2: The cells were cultured for another 1 day using Advanced RPMI-1640 medium containing 200 ng/mL FGF9, 50 ng/mL BMP7 and 100 nM vitamin A as the inducing culture medium.

S3: The cells obtained in S2 were cryopreserved by the following process: discarding cell culture medium, gently rinsing the cells with 1 mL of PBS without calcium ions and magnesium ions at room temperature, adding Accutase enzyme to the plate at 1 mL/well, digesting in a 37 °C incubator for 2 minutes, then gently blowing the digested cells off from the bottom of the well using a pipette, transferring 1 mL of cell suspension to a 15 mL centrifuge tube, adding 10 mL of DMEM/F12 medium containing 10% (v/v) fetal bovine serum, gently and evenly mixing, then centrifuging at 200×g for 4 minutes at room temperature, discarding the supernatants, resuspending the cell pellet with a cell cryopreservation solution, then transferring the cells to a cryopreservation tube, placing the cells in a -80 °C refrigerator after gradient freezing, and then transferring the cells to a liquid nitrogen tank for cryopreservation on the next day.

S4: 1 mL of type I collagen was added to a 6-well plate, and the plate was incubated in a 37 °C incubator for at least 1 hour for later use. The cells cryopreserved in S3 were taken out from the liquid nitrogen tank, quickly recovered in a 37 °C water bath, and then the cell suspension was transferred to a 15 mL centrifuge tube. 10 mL of DMEM/F12 medium containing 20% (v/v) fetal bovine serum was added, and then the obtained mixture was centrifuged at 200×g for 4 minutes at room temperature.

S5: The cells were resuspended with DMEM/F12 medium containing 20% (v/v) fetal bovine serum and Glutamax-1 (100×), and the cells were seeded in the 6-well plate coated with type I collagen at a density of 5×10⁶ cells/well. Vitamin D3 with a working concentration of 100 nM and vitamin A with a working concentration of 100 nM were added. The culture medium was changed every 24 hours.

The cells after recovery were in poor condition and could not grow. Apoptosis occurred 4 days after recovery (Figure 8).

In addition, the cells in step S2 were cryopreserved before completely differentiating into nephron progenitor cells. After recovery, the cells were cultured continuously with the culture medium in step S2 (Advanced RPMI-1640 medium containing 200 ng/mL FGF9, 50 ng/mL BMP7 and 100 nM vitamin A), but could not grow. Apoptosis occurred after four days. The experimental results show that the recovered cells are in poor condition and cannot grow if the cells that are not fully differentiated into nephron progenitor cells are cryopreserved, since they are still undergoing differentiation into nephron progenitor cells.

### Comparative Example 2

The method for preparing podocytes in this comparative example was different from that in Example 1 in that vitamin A was added to the initiating culture medium in step S1 and was not added to the inducing culture medium in step S2. The specific process was as follows.

S1: A single layer of pluripotent stem cells were continuously induced for 3 days using Advanced RPMI-1640 medium containing 5 µM CHIR99027 and 100 nM vitamin A as the initiating culture medium, and the culture medium was changed every 24 hours.

S2: The cells were continued to be cultured for 2 days using Advanced RPMI-1640 medium containing 200 ng/mL FGF9 and 50 ng/mL BMP7 as the inducing culture medium, and the culture medium was changed every 24 hours.

S3: Some of the cells obtained in step S2 were taken to validate the expressions of pluripotent stem cell markers (*OCT4* and *NANOG*) and nephron progenitor cell markers (*WT1* and *PAX2*) by qPCR. The expressions were compared to determine the effect of the induction to nephron progenitor cells.

Some of the cells obtained in step S2 were cryopreserved by the following process: discarding cell culture medium, gently rinsing the cells with 1 mL of PBS without calcium ions and magnesium ions at room temperature, then adding Accutase enzyme to the plate at 1 mL/well, digesting in a 37 °C incubator for 2 minutes, then gently blowing the digested cells off from the bottom of the well using a pipette, transferring 1 mL of cell suspension to a 15 mL centrifuge tube, adding 10 mL of DMEM/F12 medium containing 10% (v/v) fetal bovine serum, gently and evenly mixing, then centrifuging at 200×g for 4 minutes at room temperature, discarding the supernatants, resuspending the cell pellet with a cell cryopreservation solution, then transferring the cells to a cryopreservation tube, placing the cells in a -80 °C refrigerator after gradient freezing, and then transferring the cells to a liquid nitrogen tank for cryopreservation on the next day.

S4: 1 mL of type I collagen was added to a 6-well plate, and the plate was incubated in a 37 °C incubator for at least 1 hour for later use. The nephron progenitor cells cryopreserved in S3 were taken out from the liquid nitrogen tank, quickly recovered in a 37 °C water bath, and then the cell suspension was transferred to a 15 mL centrifuge tube. 10 mL ofDMEM/F12 medium containing 20% (v/v) fetal bovine serum was added, and then the obtained mixture was centrifuged at a centrifugal force of 200 g for 4 minutes at room temperature.

S5: The cells was resuspended with DMEM/F12 medium containing 20% (v/v) fetal bovine serum and Glutamax-1 (100×), and the cells were seeded in the 6-well plate coated with type I collagen at a density of 5×10⁶ cells/well after the excessive type I collagen was removed. Vitamin D3 with a working concentration of 100 nM and vitamin A with the working concentration of 100 nM were added. The culture medium was changed every 24 hours. Cell apoptosis occurred 4 days after recovery

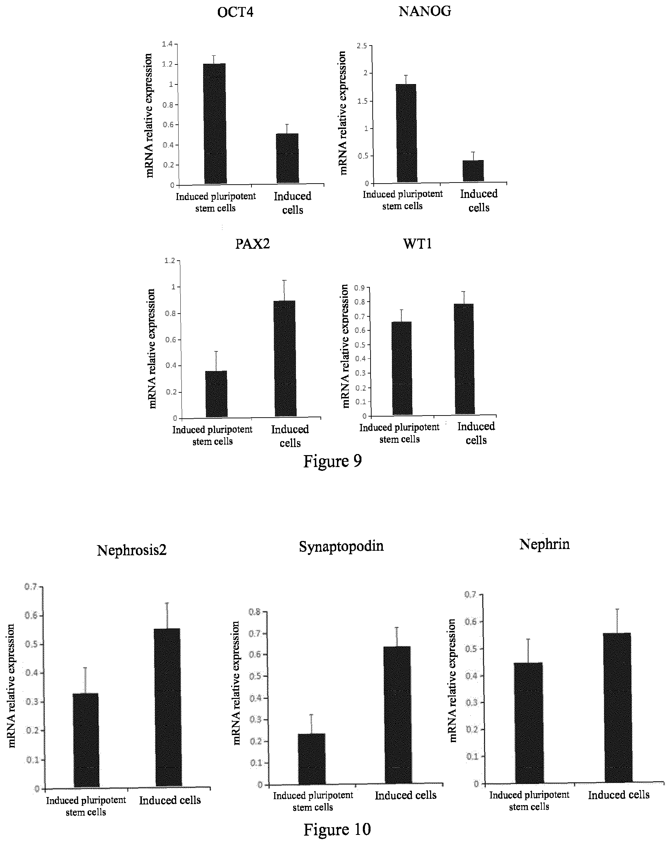

Results: The qPCR results are shown in Figure 9. Compared with the pluripotent stem cells, the expressions of genes *OCT4* and *NANOG* of the cells in step S2 decreased slightly, and the expressions of genes *PAX2* and *WT1* increased. The cells in step S2 failed to fully differentiate into nephron progenitor cells.

### Comparative Example 3

The method for preparing podocytes in this comparative example was different from that in Example 1 in that vitamin A in S5 was replaced with retinoic acid. The specific process was as follows.

S 1: A single layer of pluripotent stem cells were continuously induced for 3 days using Advanced RPMI-1640 medium containing 5 µM CHIR99027 as the initiating culture medium, and the culture medium was changed every 24 hours.

S2: The cells were cultured for another 2 days using Advanced RPMI-1640 medium containing 200 ng/mL FGF9, 50 ng/mL BMP7 and 100 nM vitamin A as the inducing culture medium, and the culture medium was changed every 24 hours.

S3: The cells obtained in S2 were cryopreserved by the following process: discarding cell culture medium, gently rinsing the cells with 1 mL of PBS without calcium ions and magnesium ions at room temperature, then adding Accutase enzyme to the plate at 1 mL/well, digesting in a 37 °C incubator for 2 minutes, then gently blowing the digested cells off from the bottom of the well using a pipette, transferring 1 mL of cell suspension to a 15 mL centrifuge tube, adding 10 mL of DMEM/F12 medium containing 10% (v/v) fetal bovine serum, gently and evenly mixing, then centrifuging at 200×g for 4 minutes at room temperature, discarding the supernatants, resuspending the cell pellet with a cell cryopreservation solution, then transferring the cells to a cryopreservation tube, placing the cells in a -80 °C refrigerator after gradient freezing, and then transferring the cells to a liquid nitrogen tank for cryopreservation on the next day.

S4: 1 mL of type I collagen was added to a 6-well plate, and the plate was incubated in a 37 °C incubator for at least 1 hour for later use. The nephron progenitor cells cryopreserved in S3 were taken out from the liquid nitrogen tank, quickly recovered in a 37 °C water bath, and then the cell suspension was transferred to a 15 mL centrifuge tube. 10 mL ofDMEM/F12 medium containing 20% (v/v) fetal bovine serum was added, and then the obtained mixture was centrifuged at 200×g for 4 minutes at room temperature.

S5: The cells was resuspended with DMEM/F12 medium containing 20% (v/v) fetal bovine serum and Glutamax-1 (100×), and the cells were seeded in the 6-well plate coated with type I collagen at a density of 5×10⁶ cells/well. Vitamin D3 with a working concentration of 100 nM and retinoic acid with a working concentration of 100 nM were added. The culture medium was changed every 24 hours, and the cells continued to be cultured for 8 days.

The cells were collected, and the expressions of podocyte marker genes *Nephrosis2, Synaptopodin* and *Nephrin* were measured by qPCR to determine the induced differentiation effect on the cells.

Results: as shown in Figure 10, there was no significant difference in the expressions of genes *Nephrosis2* and *Nephrin* between the human induced pluripotent stem cells and the cells obtained in step S5 (P >0.05). Therefore, it can be determined that the cells in step S5 failed to fully differentiate into podocytes.

The embodiments of the present disclosure have been described in detail above in conjunction with the attached drawings. However, the present disclosure is not limited to the above embodiments. Various modifications can also be made without departing from the purpose of the present disclosure within the scope of knowledge possessed by those skilled in the art. In addition, without conflict, the embodiments and the features in the embodiments of the present disclosure may be combined with each other.

## Claims

1. A method for preparing podocytes from pluripotent stem cells, comprising the following steps:
S1: inducing pluripotent stem cells to an intermediate mesoderm stage using a culture medium containing a GSK-3α/β inhibitor;
S2: inducing the cells obtained in step S1 to differentiate into nephron progenitor cells using a culture medium containing FGF9, BMP7 and vitamin A; and
S3: inducing the nephron progenitor cells obtained in step S2 to differentiate into podocytes using a culture medium containing fetal bovine serum, Glutamax-1, vitamin D3 and vitamin A.

2. The method for preparing podocytes from pluripotent stem cells of claim 1, wherein:
in step S1, the GSK-3α/β inhibitor is CHIR99027, and a working concentration of the CHIR99027 is 1-100 µM;
in step S2, a working concentration of the FGF9 is 50 - 500 ng/mL, a working concentration of the BMP7 is 50 - 500 ng/mL, and a working concentration of the vitamin A is 50 - 500 nM; and
in step S3, a volume fraction of the fetal bovine serum is 5% - 30%, a volume fraction of the Glutamax-1 is 0.1% - 10%, a working concentration of the vitamin D3 is 50 - 500 nM, and a working concentration of the vitamin A is 50 - 500 nM.

3. The method for preparing podocytes from pluripotent stem cells of claim 1, wherein:
a culture time for inducing in step S1 is 65 - 96 hours;
a culture time for inducing the cells obtained in step S1 to differentiate into nephron progenitor cells in step S2 is 42 - 72 hours; and
a culture time for inducing the nephron progenitor cells obtained in step S2 to differentiate into podocytes in step S3 is at least 192 hours, preferably 192 - 288 hours.

4. The method for preparing podocytes from pluripotent stem cells of claim 1, wherein the pluripotent stem cells are human induced pluripotent stem cells.

5. The method for preparing podocytes from pluripotent stem cells of claim 1, wherein:
the culture medium in step S1 is Advanced RPMI-1640 medium;
the culture medium in step S2 is Advanced RPMI-1640 medium; and
the culture medium in step S3 is DMEM/F12 medium.

6. The method for preparing podocytes from pluripotent stem cells of claim 1, wherein:
before inducing the nephron progenitor cells to differentiate into podocytes in step S3, the nephron progenitor cells are transferred to a collagen-coated culture vessel.

7. The method for preparing podocytes from pluripotent stem cells of claim 1, wherein:
before inducing the pluripotent stem cells to an intermediate mesoderm stage using a culture medium containing a GSK-3α/β inhibitor, the pluripotent stem cells are cultured in a culture medium containing a ROCK inhibitor.

8. A method for preparing podocytes from nephron progenitor cells, wherein the nephron progenitor cells are induced to differentiate into the podocytes using a culture medium containing fetal bovine serum, Glutamax-1, vitamin D3 and vitamin A.

9. A method for discontinuous differentiation of pluripotent stem cells into podocytes, comprising the following steps:
S1: inducing pluripotent stem cells to an intermediate mesoderm stage using a culture medium containing a GSK-3α/β inhibitor;
S2: inducing the cells obtained in step S1 to differentiate into nephron progenitor cells using a culture medium containing FGF9, BMP7 and vitamin A;
S3: cryopreserving the nephron progenitor cells obtained in step S2;
S4: recovering the nephron progenitor cells cryopreserved in step S3; and
S5: inducing the nephron progenitor cells recovered in step S4 to differentiate into podocytes using a culture medium containing fetal bovine serum, Glutamax-1, vitamin D3 and vitamin A.

10. Podocytes prepared by the method of any one of claims 1 to 9.
